# EUROPEAN PATENT APPLICATION

(11) **EP 4 796 210 A2**
(43) Date of publication of application: **26.08.2026**
(21) Application number: 26190981.6
(22) Date of filing: 23.09.2022
(51) Int. Cl.: B01F 101/20

(54) **MIXING DEVICE OF AT LEAST TWO COMPOUNDS**

(30) Priority: 27.09.2021 IT 202100024638
(62) Document number(s) of the earlier application(s) in accordance with Art. 76 EPC: 22802703.3 / 4 408 575
(71) Applicant: TECRES S.P.A., 37066 Sommacampagna (VR) (IT)
(72) Inventor: FACCIOLI, Giovanni, 37066 Sommacampagna (Verona) (IT); SOFFIATTI, Renzo, 37066 Sommacampagna (VR) (IT)
(74) Representative: Feltrinelli, Secondo Andrea

(57) **Abstract**

The present invention relates to a mixing device for at least two compounds, such as a liquid and a powder for obtaining bone cement, as well as a method of mixing at least two compounds using such mixing device.

## Description

### TECHNICAL FIELD OF THE INVENTION

The present invention relates to a mixing device for at least two compounds, such as a liquid and a powder for obtaining bone cement, as well as a method of mixing at least two compounds using such mixing device.

### STATE OF THE ART

Many types of liquid-powder mixers have been proposed for obtaining bone cement.

The Italian patent no. 1236864 as well as the European patent no. 1912597, both in the name of the applicant of the present patent application, teach a mixer comprising a first component delimiting a powder containment chamber, in which a second component is slidingly mounted defining a housing zone for a vial containing a liquid to be mixed with the powder.

A stem supporting a mixing component is then slidably mounted in the powder containment chamber, while a cap delimiting an annular groove for housing a gasket is mounted on the internal, in use, end of the second component, the internal end of the cap delimiting a shoulder facing away from the containment chamber and intended to abut against a respective shoulder of the first component.

With these devices, the vial is broken, the second component is moved manually so as to come out of the first component, thus causing the liquid of the broken vial to enter the powder containment chamber, and then mixing is carried out at the same time inside of the latter until the bone cement is obtained. If desired, during the movement of the second component, a vacuum is applied to the powder containment chamber to facilitate the transfer of the liquid into the latter.

With these devices it is very difficult to achieve a good seal in the powder containment chamber, taking into account that the gasket, having to allow the second component to be moved manually with respect to the first, cannot be excessively hard or resistant.

In this regard, during the mixing of powder and liquid, air may enter at the gasket and therefore cause the formation of bubbles in the final bone cement mixture, which, clearly, would reduce the quality of the bone cement obtained.

Moreover, the devices discussed above have a much greater volume of the first component than the vial, this in particular as a function of the configuration of the cap and of the other components of the respective device. As it will be understood, this entails, for devices according to the state of the art, an excessive final volume or width.

A further mixing device provided by the state of the art is described in European patent no. 3283206 always in the name of the applicant. This device comprises a first component and a second component in fluid communication with each other. The first component contains a first compound and the second component has a vial containing inside it, in turn, a second compound to be mixed with the first compound. Breaking means of the vial are also provided, so that the second compound can flow inside the first component to mix with the first compound, thereby obtaining a mixture.

This device has several disadvantages: the breaking of the vial, by means of the breaking means, for the discharge of the second compound takes place following crushing of the glass of the same. This could cause the deposit of glass fragments at the passage holes that the second compound uses to flow inside the first component, actually at least partially obstructing these holes. In practice, the transfer of the second compound into the first component can be considerably complicated or in any case accidentally slowed down.

A further disadvantage derives from the fact that if it were necessary to use several compounds to be mixed, and therefore more than one vial, an equal number of breaking means would consequently also be necessary, in fact, increasing the costs and manufacturing complexity of the mixing device.

Yet another disadvantage derives from the fact that the first component is made of opaque material which complicates the observation of the mixing state of the two compounds.

There is therefore a need to have a new type of mixing device of at least two compounds that overcomes the drawbacks of the known art.

### OBJECTS OF THE INVENTION

Therefore, the main object of the present invention is to improve the state of the art in the field of mixing devices of at least two compounds.

Another object of the present invention is to provide a mixing device of at least two compounds which is easy to use.

A further object of the present invention is to provide a mixing device of at least two compounds which has costs and structural complexity lower than the devices taught by the state of the art.

Still another object is to provide a mixing device which allows rapid and efficient mixing of the compounds.

Another object of the present invention is to provide a mixing device capable of mitigating the possibility that, following the breakage of containment elements of a compound, any fragments hinder the mixing of the compounds and thus the obtainment of the mixture.

According to an aspect of the present invention, a mixing device of at least two compounds is provided for obtaining a mixture according to claim 1.

According to another aspect of the present invention, a method of mixing at least two compounds is also provided for obtaining a mixture according to claim 27.

The dependent claims refer to preferred and advantageous forms of the invention.

### BRIEF DESCRIPTION OF THE DRAWINGS

Further features and advantages of the present invention will become more evident from the detailed description of some preferred embodiments of a mixing device of at least two compounds, illustrated by way of non-limiting example in the accompanying drawings in which:
- figure 1 illustrates a perspective view of a mixing device of at least two compounds according to an embodiment of the present invention;
- figures 2 and 3 show a front and side view, respectively, of a component of the device of figure 1;

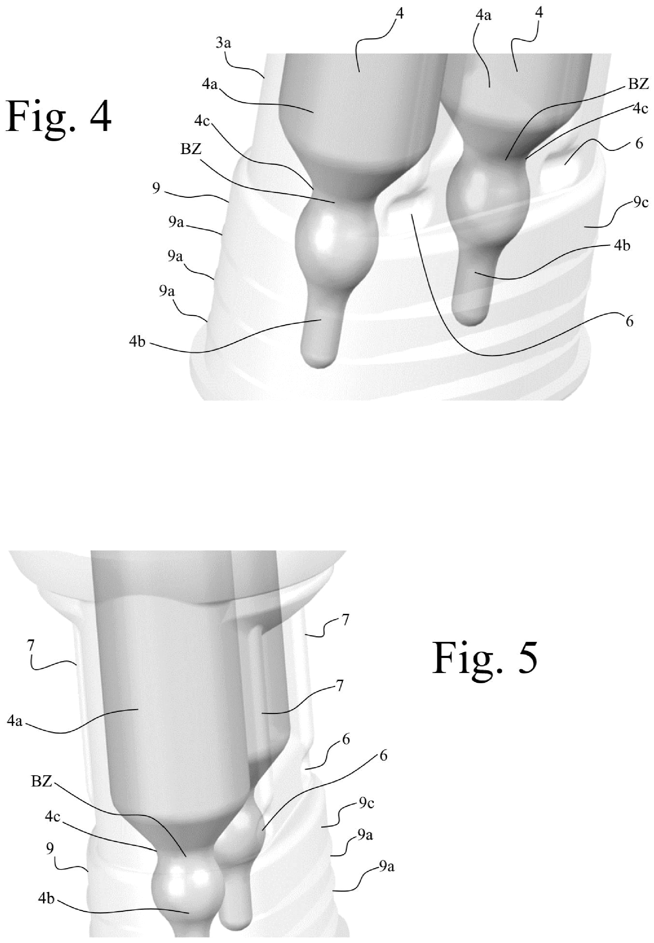

- figures 4 and 5 show details on an enlarged scale of the component of figure 2;

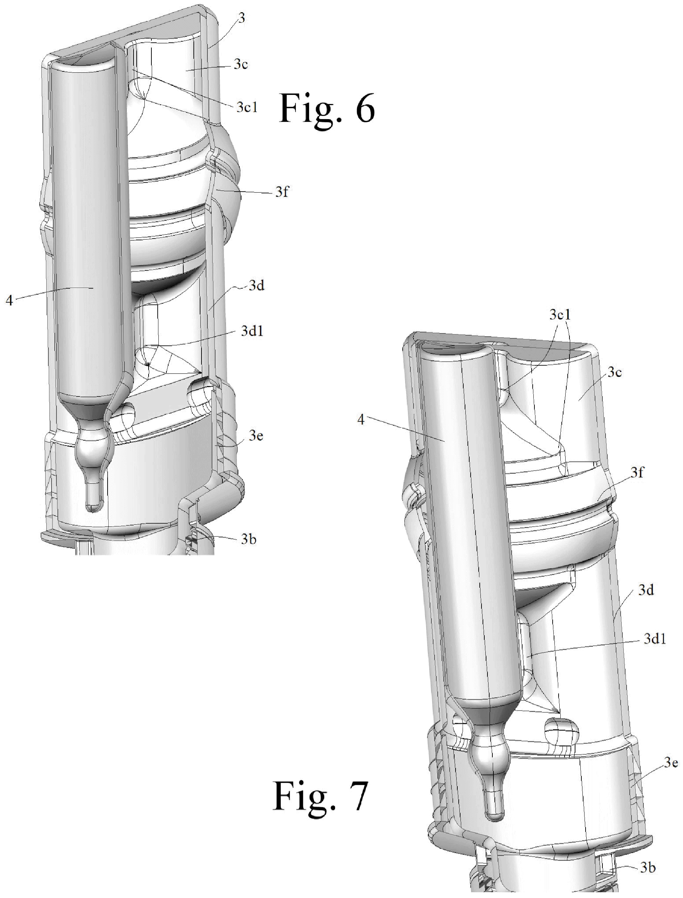

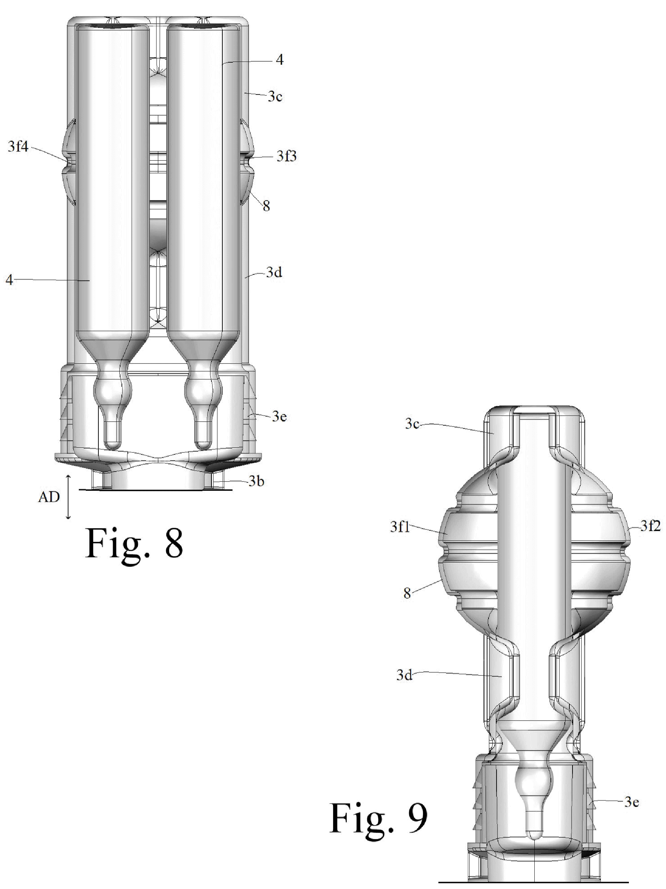

- figures 6 to 9 show respective cross-section views of the second component of the device of figure 1,

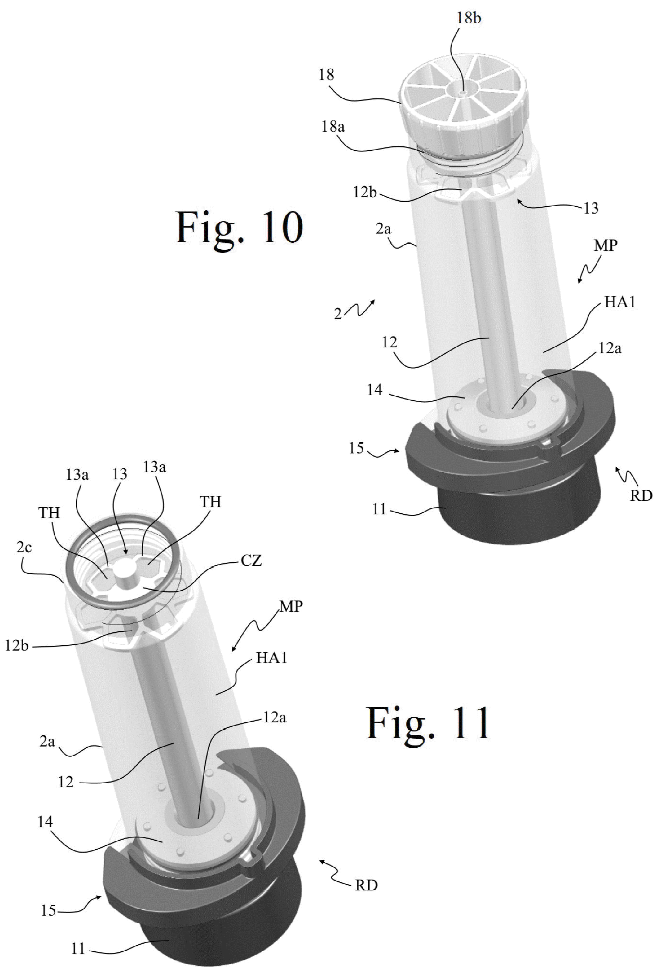

- figure 10 shows a perspective view of the first component of the device of figure 1;
- figure 11 illustrates a perspective view of the first component of figure 10 without the closure element;
- figure 12 shows a perspective view of the first component of figure 10 with removed locking means;
- figure 13 illustrates a perspective view of the first component of figure 10 operatively associated with extrusion means,
- figure 14 illustrates the mixing device of figure 1 in a folding operating configuration;
- figure 15 illustrates the mixing device of figure 1 in a release operating configuration;
- figure 16 illustrates a top view of the constraint or connection end of the second component of the mixing device of figure 1;
- figure 17 shows a sectional view of the second component of figure 6;
- figure 18 shows a front view of a detail on an enlarged scale of the device of figure 1;

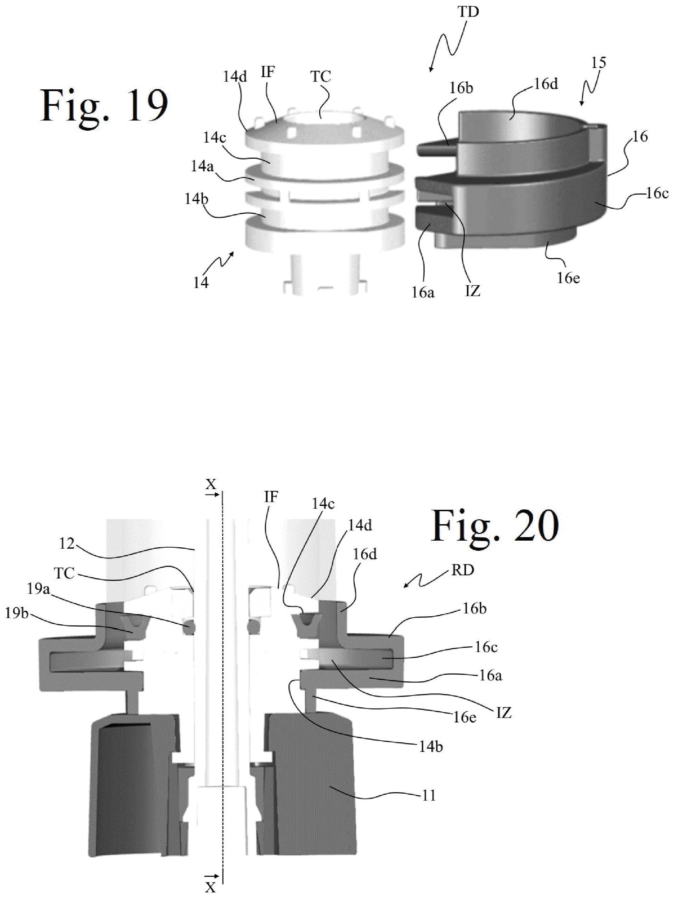

- figure 19 shows a side view of a detail on an enlarged and exploded scale of the device of figure 1;
- figure 20 shows a detail on an enlarged scale and in longitudinal section of the device of figure 1;
- figure 21 is a schematic view in longitudinal section of another embodiment of the second component for a device according to the present invention;
- figure 22 is a schematic cross-section view of the second component of figure 21, and
- figure 23 is another schematic view in longitudinal section of a further embodiment of the second component for a device according to the present invention,
- figure 24 shows a perspective view of a further embodiment of the second component for a device according to the present invention,

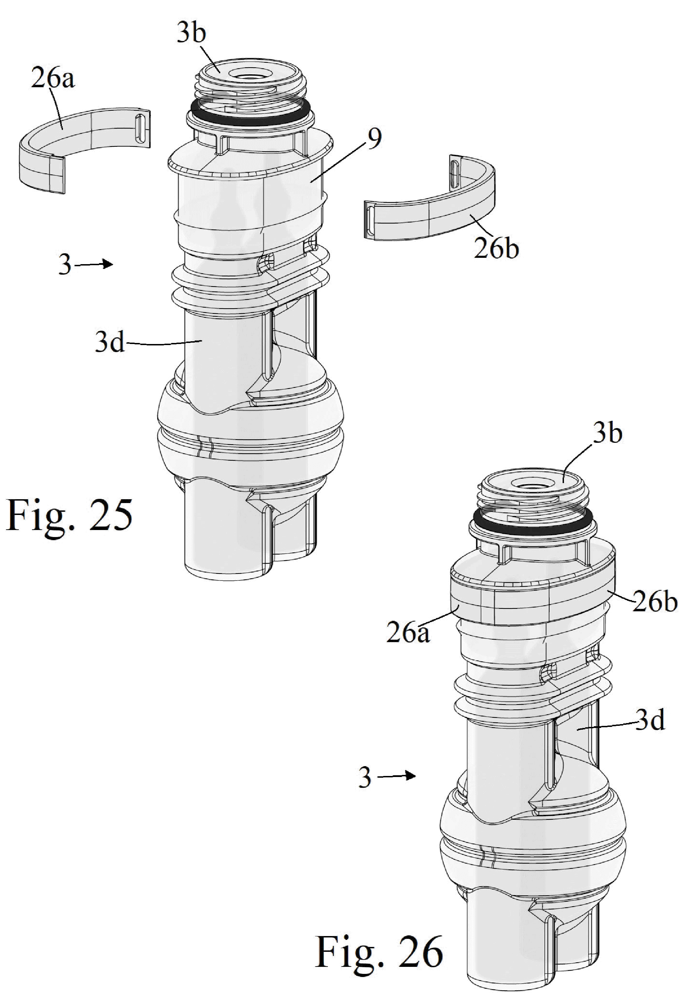

- figures 25 and 26 illustrate the second component of figure 24 with clamping means.

In the accompanying drawings, identical parts or components are indicated by the same reference numbers.

### EMBODIMENTS OF THE INVENTION

With reference to the attached figures, the reference number 1 indicates a mixing device according to the present invention for at least two compounds C1, C2 to obtain a mixture M.

The at least two compounds C1, C2 could be respectively a powder C1, such as one or more polymers, eventually acrylic, and a liquid C2, such as a monomer, for obtaining bone cement M, for example for medical uses, or an acrylic resin M.

More specifically, the powder could also be inorganic based on Calcium Salts, such as Calcium Sulphate (gypsum) or mixtures of Tricalcium Phosphate (TCP) or Hydroxyapatite (HA) or mixtures based on Calcium Oxides (CaO), Oxides of Magnesium (MgO), Aluminum Oxides (Al₂O₃), Silicon Oxides (SiO₂), Iron Oxides (Fe₂O₃) and/or other complex salts such as Calcium Metasilicate (CaSiO₃ and Ca₂Si₂O₅) and/or Tricalcium Aluminate Ca₃(AlO₃)₂ and/or Tetracalcic Ferrite Aluminate (C₄AlFe) and/or other inorganic compounds which, combined with a second compound C2, such as pure water or an aqueous solution containing inorganic salts synergistic with those present in the powder, give rise to a commonly hardening mass identifiable as "hydraulic cement".

Preferably, all compounds will possess a degree of purity such that the mixture M results in a biocompatible product or dough.

The mixing device 1 comprises a first component 2 including a first body 2a, if desired with a substantially tubular configuration at least at a respective side wall, which defines a first housing area HA1 for at least a first compound C1 and a second component 3, in use, operatively associable, for example through threaded coupling, bayonet coupling, forced fitting or other type, to the first component 2 so as to place the first and second components 2, 3 or the respective housing areas HA1, HA2 in fluid communication defining a fluid transfer axis or direction AD from the first 2 to the second 3 component.

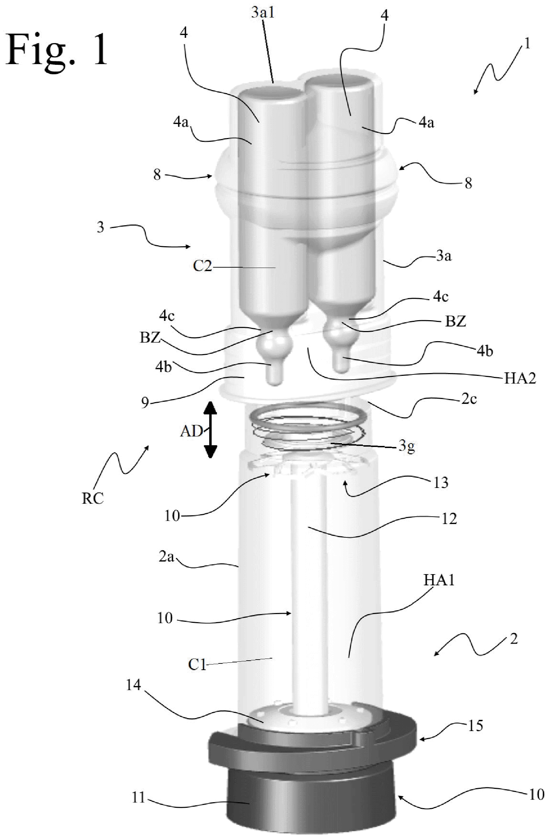

With reference to the device illustrated in figure 1, the fluid transfer axis or direction AD is substantially vertical or in any case substantially parallel to the main development axis of the first 2 and second 3 component or of the respective tubular side walls or in any case in the direction from the first 2 to the second 3 component, if desired also slightly inclined with respect to the main extension axes of the latter. Clearly, one could also have two components 2, 3 constrained inclined to each other in which case the transfer axis or direction AD could also be inclined with respect to the main development axis of one or both components 2, 3, but in any case, the axis or direction AD defines the main axis or direction along which the passage of the fluid between the two components 2, 3 takes place.

In particular, the second component 3 comprises a second body 3a, made of flexible or deformable material, if desired elastic or plastic material, i.e., elastically or plastically deformable, the second component 3 defining a second housing area HA2 for at least one second compound C2.

The second component 3 is wholly or partly translucent or transparent.

The material for making the second body 3a is thermoplastic polyurethane or TPU. Clearly, the chemical composition of the second body 3a must be such as not to absorb the second compound C2.

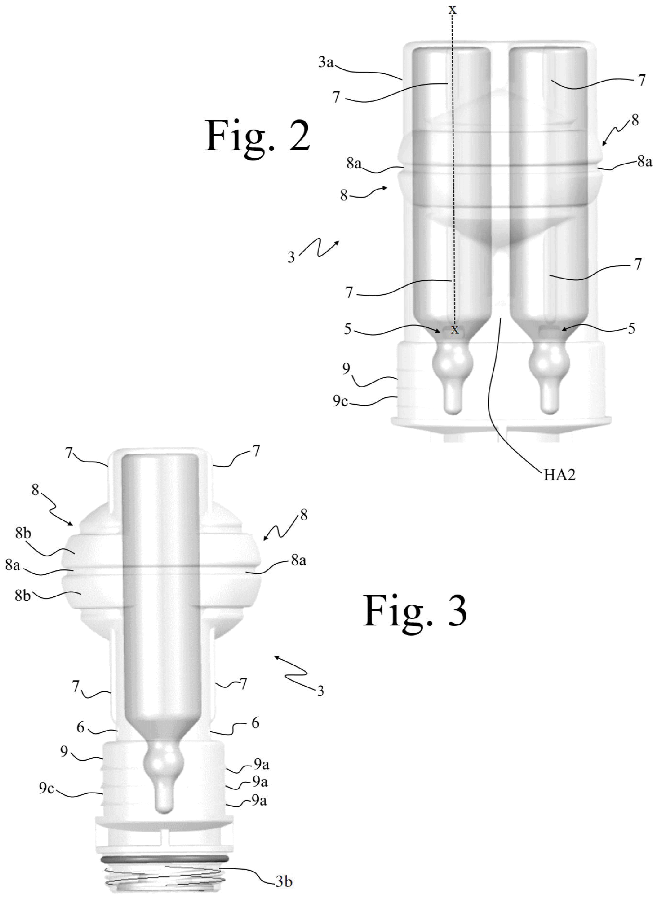

Moreover, if the second body 3a is wholly or partly translucent or transparent, as schematically shown in figures 2 and 3, it allows to observe in detail what happens inside the second body 3a during the transfer process of the at least one second compound C2 from the second housing area HA2 to the first housing area HA1, thereby giving, for example to the operator responsible for obtaining the mixture M, a high level of control over this process.

As regards the configuration of the second body 3a with reference to the non-limiting embodiment illustrated in the figures, it comprises a tubular side wall with a constraint or connection end 3b, for example internally threaded to the first component 2 which is open and defines the passage opening from the second HA2 to the first housing area HA1. The constraint or connection end 3b can be tubular and define the continuation of the side wall of the second body 3a.

The tubular wall preferably has a variable section from one end to the other.

If desired, the second body 3a is also provided with a distal, in use, wall 3a1 from the first component 2 closing the respective second housing area HA2.

Preferably but not necessarily, the second body 3a is made in one piece.

As regards the thickness of the second body 3a, it is preferably but not necessarily constant.

The first body 2a can be laterally tubular with both its ends 2b, 2c open, if desired, tubular or annular, or even with one open end only, i.e., that of connection 2c to the second component 3.

Preferably, the first body 2a is made in one piece.

The first body 2a can be advantageously made of plastic or composite material or of another material, preferably also translucent or transparent, if desired also in glass or similar materials, in order to allow the inside of the first housing area HA1 to be observed and therefore, have greater control during the mixing of the at least two compounds C1, C2 and the subsequent formation of the mixture M.

The mixing device 1 then comprises at least one containment element 4 of the at least one second compound C2, positioned or placeable within the second housing area HA2, which can include or delimit at least one breaking or opening zone or point BZ of the same.

It should be noted that the term "breaking or opening zone or point" means a zone or a point of the containment element 4 which allows, when subjected to a certain external pressure or to the action of a suitable perforator, a rapid and preferably clear, i.e., not fragmented, breaking or opening of the containment element 4 in that determined zone or point.

The at least one containment element 4 can be at least one vial, for example two or more vials, if desired made of glass or similar material, as shown in the non-limiting embodiment of the present invention of figure 1.

Preferably, the at least one containment element 4 comprises at least a containment portion of the at least one second compound C2 with for example a cylindrical body 4a and at least one pointed portion 4b, if desired intended to abut or collide against the second body 3a for opening or breaking the containment element 4.

As far as the breaking or opening zone or point BZ is concerned, it can advantageously be delimited by a narrowing 4c interposed between the cylindrical body 4a and the pointed portion, if desired, an abutment portion 4b. Clearly, no narrowing 4c may also be provided or in any case the vial 4 may not necessarily break at the same.

Alternatively, the vial 4 could have a containment body, for example made of glass, delimiting an (open) end at which a cap component mounted to close this end is provided, so as to ensure the sealing of the vial and prevent the undesired leakage of the second compound therefrom; in this regard, the cap component or a film thereof is impermeable to the second compound.

In this case, the cap component can, for example, be fixed in position on the containment body by means of a ring nut or the like, for example made of aluminum, which can be annular. The cap component, or rather a part thereof, can be pierceable; in this regard, the cap component may have a support body, for example annular, with an impermeable film to the second compound being fixed on one side, in use, internal (internal to the vial 4) of the support body, which film can be pierced by breaking or opening means of the vial.

Such a vial could be opened by means of a needle or perforator or also by means of a portion or element, for example a pointed one, of the device.

It should be noted that reference has been made to "at least one containment element 4 of at least a second compound C2", but two, if desired three, four or more containment elements 4 each containing a quantity of compound C2 or each containing different types and/or quantities of compounds could also be provided. Naturally, in general, the number of containment elements 4 positioned within the second housing area HA2 is expected to be reasonably compatible with the dimensions of the second body 3a.

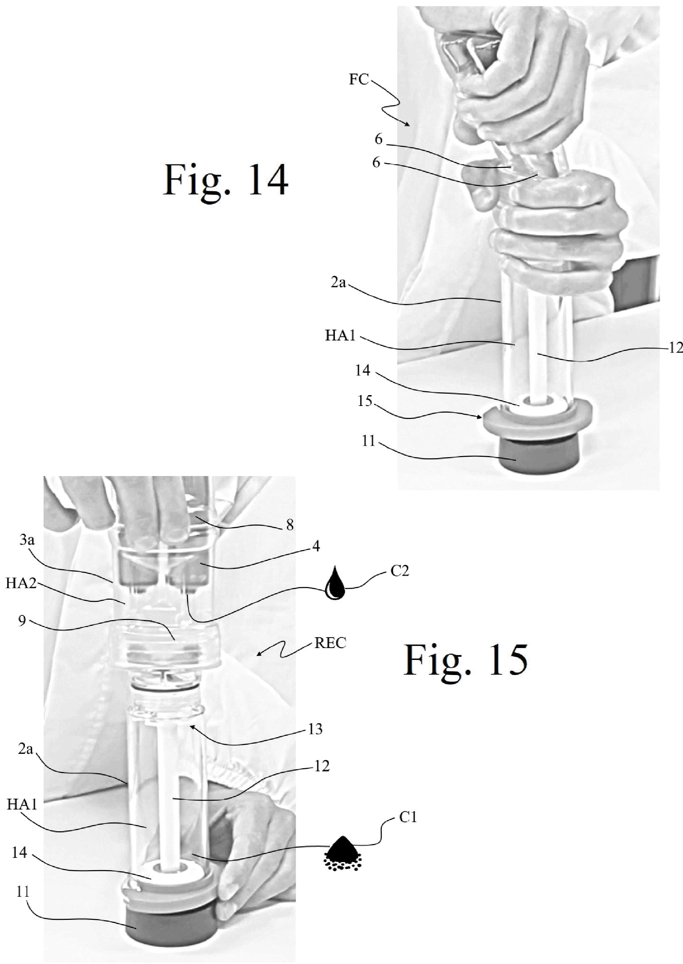

With regard to the above, the containment element 4 is displaceable or inclinable transversely or orthogonally to the transfer axis or direction AD between a first rest position (see figure 1) in which a respective abutment portion 4b is not in contact with or in thrust such to determine its breaking or opening against the second component 3 or with elements of the device integral with it and at least one second breaking or opening position (see figure 14) in which the containment element 4 or one or more respective abutment portion 4b impacts against the second component 3 or presses against the same (against a respective internal portion) or with an element of the device integral therewith to a greater extent than the first position.

With specific reference to the rest position, the second component 3 or a respective abutment portion 4b can therefore not be in contact with other elements (second component 3) of the device or it can also be in contact with them, but not in thrust upon them or in any case in thrust not such as to cause the breaking or opening of the containment element 4.

In the passage from the first to the second position, following the impact, the containment element 4 breaks or opens in such a way that the second compound C2 inside it can exit from the containment element 4 and pass or flow from the second housing area HA2 to the first housing area HA1 along the fluid transfer axis or direction AD. This passage can be obtained by gravity, by placing the second component 3 in a position higher than the first component 2 and/or by pressurizing or pumping the second component 3 or, if desired, by suction at the first component 2.

With reference to the expression transversely or orthogonally to the transfer axis or direction AD, it has been observed that the transfer direction AD is a direction of passage from the second HA2 to the first HA1 area, thus an approach zone to the first component 2 from the second HA2, which, with reference for example to figure 1, is actually a vertical axis or direction.

Therefore, the fact that the containment element 4 is movable or inclinable transversely or orthogonally to the transfer axis or direction AD, implies that the containment element 4 is broken or opened if it is moved at least in part along a direction which is not that of transfer AD.

Basically, in order to be opened or broken, the containment element 4 is not pushed, as occurs according to the state of the prior art, towards the first component, but is inclined, from one side to the other, or moved horizontally or orthogonally or transversely with respect to the axis or direction AD, so as to bring a respective abutment portion, into contact or impact against specific portions of the second component 3 or also against protruding portions or elements, preferably internally of the device 1 and, if desired, in one piece with the second component 3. Clearly, the displacement or inclination direction of the containment element 4 may also include (but not only) an approach component to the first component 2.

In this regard, it is clearly important that the containment element 4 is displaced relative to the component or element of the device on which or against which it must impact or must contact, such as for example a respective portion 3e of the second component 3 or of an element integral therewith.

Preferably, as partly indicated, the second component 3 has a constraint or connection end 3b to the first component 2 defining or extending around the fluid transfer axis or direction AD and at least one section projecting (consisting of one or more portions or sections 3c, 3d, 3f of the respective tubular wall) from the first end 3b, which projecting section is inclinable or foldable with respect to the fluid transfer axis or direction AD, so that by determining the inclination or bending of the projecting section with respect to the first end 3b, one cause the inclination of the containment element 4 mounted within the second component 3 and therefore the passage of the same between the first and the at least one second position and the respective breaking or opening thereof.

In this regard, the containment element 4 is preferably mounted within the second component 3 and integral in displacement or inclination with it or better with the at least one respective projecting section 3c, 3d, 3f.

Preferably, the second component 3 has, from one end to the other, several tubular sections, one with a section different from the others, designed to internally define a specific face or internal surface defining the second housing area HA2 and, if desired, clearly also a corresponding external configuration.

In this regard, one, two or more sections 3c, 3d of the second component 3 for retaining the containment element 4 can be provided, which or whose sections 3c, 3d are designed to clamp or surround or engage a respective part of the containment element 4 so as to keep it in a specific position relative to another component of the device, for example at a distance from the connection end 3b or in any case with a respective abutment or collide portion 4b in a specific internal zone defined by component 3.

A section 3e for receiving with slack or play a respective abutment portion 4b, for example the tip of the containment element 4, can then be provided, which section 3e is preferably proximal, in use, to the first component 2 or in any case adjacent and subsequent to the constraint end 3b of the second component 3 to the latter. Clearly, the maintenance of the abutment or collide portion 4b in the zone or area defined by the section 3e is specifically delegated to the presence of one or more holding sections 3c, 3d. Moreover, the containment element 4 is preferably not integral in displacement or inclination with the receiving section 3e, so the receiving element can be moved or inclined with respect to the latter.

The holding sections 3c, 3d can have any suitable section, for example ellipsoidal or rectangular (if desired with rounded edges), if desired with one or more intermediate narrowings.

The receiving section 3e can have any suitable section, for example ellipsoidal or rectangular (if desired with rounded edges), if desired without one or more narrowings.

With reference to this, each holding section 3c, 3d can be internally defined by respective projections or protuberances or recesses 3c1, 3d1 designed to engage, for example to size, if desired with the possibility of sliding following an appropriate thrust a respective portion of the containment element 4. Clearly, the internal face of the sections 3c, 3d could be configured as the entire perimeter of a tubular body of the containment element 4, so as to completely engage the latter perimeter or they could be such as to engage only portions of circumference of the tubular body of the containment element 4.

On the other hand, the receiving section 3e with slack can instead have an internal section considerably greater than the containment element 4 or of the respective containment elements of a tip 4b of the same, for example greater than 3/4 or 3/2 of the volume of said tip or tips 4b, even if it is desired three, four, five or more times greater than the volume of the latter and in any case the portion 4b of the containment elements 4 arranged inside the area or zone defined by the receiving section 3 is not clamped by the latter and is free to move or tilt in the passage from the first to the second position, until, for example, it abuts against a portion of the internal surface of the section 3e or of another component of the device 1.

The second component 3 can then comprise, if desired adjacent and subsequent to a holding section or between two possible holding sections 3c, 3d, a pumping section 3f which will be described more fully later on. The pumping section 3f can have any suitable section, for example circular or ellipsoidal, with variable diameter or section, for example first increasing and then decreasing, approaching the connection end 3b.

With regard to the now proposed description of the second component 3, the at least one projecting section which can be inclined or bent with respect to the fluid transfer axis or direction AD, it is preferably constituted by the at least one holding section 3c, 3d and by the pumping section 3f, while the receiving section 3e, in the passage from the first to the second position, remains substantially integral with the constraint or connection end 3b, although it could move slightly.

With reference to the non-limiting embodiment illustrated in the figures, two containment elements or vials 4 are provided, and the internal surface 3a of the second component 3 is configured in such a way as to hold, for example side by side, if desired with a space between them, the two or more containment elements 4.

In this regard, one or more of the sections of the second component 3 could internally define projections or protuberances or recesses actually designed to separate the positioning zone of each containment element 4.

Preferably, the possible containment elements 4 would then be movable or inclinable together or simultaneously, acting on them or externally on the second component 3.

Advantageously, the second component 3 comprises folding means 5 configured to allow or facilitate the folding of at least a portion or section 3c, 3d, 3f of the second body 3a from the first to the second position, at such folding means 5, so as to determine the breaking of the at least one containment element 4, if desired the narrowing 4c at or around the breaking zone or point BZ so that the at least one second compound C2, in use, passes or flows (or rather can pass or flow) from the second housing area HA2 to the first housing area HA1.

It should be noted that, in the present description, the folding or non-folding of any component of the mixing device 1 is to be considered with reference to the usual pressure ranges to which it can be subjected during the operations for obtaining the mixture M, such as for example the folding force of an operator or the folding force predetermined by an automatic system designed to carry out the operations necessary to obtain the mixture M.

Preferably, the folding means 5 are or comprise at least one weakening element or at least one weakening portion 6, for example at least one depression or hollow, delimited in the second body 3a so that, following the folding of the latter or of respective sections, it folds at the at least one weakening element or portion 6 to cause the breaking of the at least one containment element 4 or, more particularly, of the narrowing 4c at or around the breaking zone or point BZ.

According to the non-limiting embodiment of the present invention shown in the figures, the at least one weakening element or portion 6 consists of two, three, four or more depressions 6 delimited in the second body 3a and positioned offset from each other, if desired two by two, one opposite to the other with respect to a longitudinal section plane of such second body 3a.

These depressions 6 can be provided for or are at the receiving section 3e or rather between a holding section 3d and the receiving section 3e.

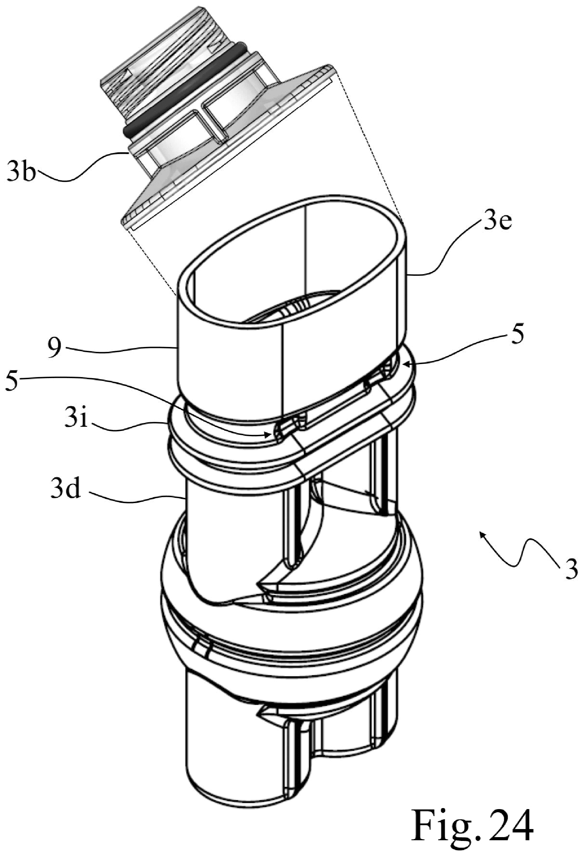

Moreover, the second component 3 or, more particularly, the second body 3a can advantageously comprise, as shown for example in figure 24, a bellows element or section 3i arranged between the holding section 3d and the receiving section 3e, if desired arranged in proximity to the folding means 5, if provided. This bellows element or section 3i is designed to facilitate the folding of the second body 3a so as to simplify or in any case improve the process of obtaining the mixture M.

The second component 3 or, more particularly, the second body 3a can advantageously comprise or delimit also one or more stiffening elements or sections 7, preferably extending along a longitudinal direction x-x of the second component 3 and configured to stiffen the second body 3a so that the latter, in use, does not fold at such one or more stiffening elements or sections 7.

According to the non-limiting embodiment of the present invention shown in the figures, a stiffening element or section 7 is provided, which extends along a longitudinal direction x-x of the second body 3a, in proximity to each folding means 5 or, more in particular, of each weakening element or portion 6 or even more in detail of each depression 6.

These stiffening portions 7 can be provided or are at one or more holding sections 3c, 3d.

Preferably, the second component 3 also comprises pumping means 8 configured, in use, to speed up the release or in any case facilitate the transfer of the at least one second compound C2 from the at least one containment element 4 to the second housing area HA2 once that the containment element 4 has been broken or opened.

The pumping means 8 can advantageously be or comprise one or more enlarged or balloon-shaped or raised portions 8a delimited in the second body 3a, if desired having one or more shapes 8b, for example annular.

According to the non-limiting embodiment of the present invention shown in the figures, the pumping means 8 consist of one, two or more enlarged or balloon-shaped portions 8a delimited in the second body 3a.

With reference to the non-limiting example of embodiment illustrated in the figures, the pumping section 3f actually defines the pumping means 8.

In this regard, the pumping section 3f has at least two main segments 3f1, 3f2 opposite each other at a distance greater than the width of a respective containment element 4, so that there is a pumping area PA between the latter and the two main segments 3f1, 3f2 once the at least one containment element 4 is housed in the second housing area HA2.

If desired, the pumping section 3f, considering the respective perimeter, also has one or two side segments 3f3, 3f4 for connecting the first two segments 3f1, 3f2 and which are at a distance from each other equal to the width of the containment elements 4 (one, two or more) provided in the device, with possible interposition of projections or protuberances or recesses.

In this regard, the pumping section 3f can also have a circular configuration, so that the various segments 3f1-3f4 are equidistant from a respective center, but the containment elements 4 are mounted side by side, so as to result each with a respective end at close to a portion or segments 3f3, 3f4 of the pumping section 3f, but with at least one respective pumping area or space between the containment elements 4 and other portions or segments 3f1, 3f2 of the pumping section 3f.

Basically, the containment element 4, in the pumping section 3f, in a rest or not-pumping condition of the latter, is not in contact with at least one and preferably with both the two main segments 3f1, 3f2 and, if provided, in contact or in any case close to the two side segments 3f3, 3f4.

In particular, following the pressure and subsequent release of the pumping means 8 and more particularly of the second body 3a at one or both of the main segments 3f1, 3f2, it is possible to increase and subsequently decrease the air pressure in the second housing area HA2 and therefore, of the air in the part of the latter housing the breaking zone or point BZ so as to accelerate the release of the at least one second compound C2 contained in the at least one containment element 4 or in any case facilitate the transfer of the at least one second compound C2 from the at least one containment element 4 to the second housing area HA2, clearly after the at least one containment element 4 has been broken or opened.

Basically, since the air is a compressible fluid, the pressure and the subsequent release of the pumping means 8 cyclically decreases and increases the volume for the air present inside the second housing area HA2, generating in the latter an alternating increase and a decrease of the pressure, thus determining a faster emptying of the containment element 4 and the quicker passage of the respective second compound 2 into the first housing area HA1.

It should be noted that, if the second component 3 comprises pumping means 8 or, more particularly, one or more enlarged or balloon-shaped portions 8a, the one or more stiffening elements or sections 7, if provided, can stop at said pumping means 8 and then go on or in any case continue to extend, for example along the same longitudinal direction x-x.

As regards the positioning of the at least one containment element 4 inside the second housing area HA2, the second component 3, or more particularly, the second body 3a can advantageously define, depending on the configuration of the internal face, in particular at the holding section or sections 3c, 3d, at least one positioning seat PS configured to support the at least one containment element 4.

Preferably, the at least one containment element 4 is engaged in the at least one positioning seat PS by means of forced or to size fitting, so that it is not necessary to use separate or specific support means, such as springs or the like separated from the second body 3a, for the at least one containment element 4.

In practice, the at least one containment element 4 can be engaged in the at least one positioning seat PS which, thanks to its particular configuration, is able to self-support it without using additional components.

With regard to this aspect, the at least one containment element 4 is supported at a distance from the engagement zone between the first 2 and the second 3 component, so that the respective tip 4b is, for example, in the receiving section 3e with slack.

As indicated above, the second component 3 can then advantageously comprise or delimit a constraint or connection end 3b, for example internally threaded or for the bayonet or plug-in engagement to the first component 2.

In particular, this constraint or connection end 3b defines at least one through opening TO so as to allow, in use, the passage or transfer of the at least one second compound C2 from the second housing area HA2 to the first housing area HA1.

Moreover, in the section wall 9 of the second component 3, which actually defines the receiving portion 3e with slack or play and which is, if desired, adjacent to the constraint or connection end 3b, the second component 3 preferably comprises or delimits reinforcement elements or sections 9a configured to reinforce this wall section 9 so that, in use, it does not fold.

Advantageously, the second body 3a has stiffeners such as to drive the possible folding of the latter not at them, but for example at any folding means 5 or more in particular, at the at least one weakening element or portion 6.

The device 1 can also comprise one or more retaining elements 3h at the end 3b, which retaining elements 3h are for example positioned at the at least one through opening TO, and are configured to retain, in use, the broken parts of the at least one containment element 4 inside the second housing area HA2.

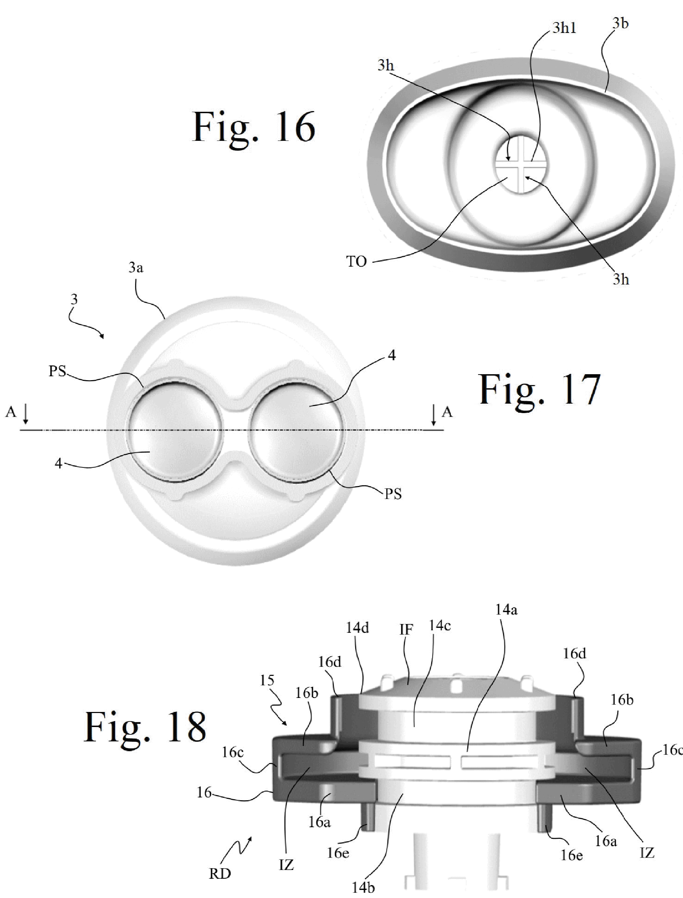

The one or more retaining elements 3h, according to the non-limiting embodiment shown in figure 16, are a cross-shaped element 3h1, but clearly in other embodiments they could also be an element configured as a network or grid.

Advantageously, a filter 3g is also provided to intercept the passage opening or light TO delimited by the connection end 3b of the second component 3, which can serve, among other things, to prevent the second compound from passing backwards from the first HA1 to the second housing area HA2.

Advantageously but not necessarily, the breaking of the at least one containment element 4 preferably occurs by substantially clean cut so that no fragments are released which could accidentally obstruct the passage from the second HA2 to the first housing area HA1 and ruin the obtainment of the mixture M.

It should be noted that the constraint or connection end 3b can be made either in one piece with the second body 3a, or even separately and subsequently connected immovably or not to this second body 3a.

In this regard, see for example the non-limiting embodiment of the second component 3 shown in figure 24, in which the wall section 9 is shown in one piece with the second body 3a, while the constraint or connection end 3b, or in any case the component or the portion constituted by it, can be removably constrained, for example by means of forced fitting, screwing, bayonet engagement or in any case by means of a suitable removable constraint, to this wall section 9.

With regard to this aspect, this could, for example, allow the use of different constraint or connection ends 3b to adapt, if necessary, the constraint or connection of the second component 3 to any dimensions or measurements of the connection end 2c of the first component 2.

According to the non-limiting embodiment illustrated in the figures, the constraint or connection end 3b comprises an annular shank section, if desired externally threaded and extending from a base section that can be constrained (in any suitable way) to the wall section 9, which annular shank section is designed to engage the connection end 2c by screwing or interlocking or in another suitable way.

With regard to this aspect, safely clamping means 26a, 26b of the constraint or connection end 3b to the wall section 9 could also be provided, designed to preferably removably constrain these components.

The clamping means, if provided, may include for example a locking ring, if desired but not necessarily made with two C-shaped or jaw components 26a, 26b that can be removably constrained to each other at their respective free ends. With reference to this aspect, the ends of the two C-shaped or jaw components are engageable in any suitable way, such as by interlocking, fitting or by constraint between one or more teeth on the ends of a component 26a and/or one or more holes or slots on the ends of the other component 26b.

As it will be understood, in order to securely tighten the constraint or connection end 3b to the wall section 9, once these components have been assembled, they will be engaged by means of the clamping means 26a, 26b, for example by encircling part of the components 3b, 9 assembled with the two C-shaped or jaw components 26a, 26b, which will then be engaged as indicated above at their respective ends and placed one opposite the other with respect to the assembled components 3b, 9.

Owing to the safely clamping means 26a, 26b it is therefore possible to prevent the undesired separation of the two parts 3b and 9, clearly if they are not in one piece.

Again, with reference to the non-limiting embodiment illustrated in the figures, the second component 3 operatively associated, in use, with the first component 2 preferably comprises at least three operating configurations RC, FC, REC.

The at least three operating configurations RC, FC, REC include at least one rest operating configuration, in which the body 3a is not folded and the at least one containment element 4 is not broken, at least one folding operating configuration FC, in which the operating body second body 3a is folded, if it is desired at the folding means 5 and at the at least one containment element 4 to collide with the second body 3a thus causing the breaking of the at least one containment element 4 or more particularly, of the narrowing 4c, at or around the breaking zone or point BZ and at least one release operating configuration REC in which the second body 3a is not folded or is folded less than the folding configuration FC and the at least one containment element 4 or, more in in particular, the narrowing 4c is broken at or close to the breaking zone or point BZ so as to allow the release of the at least one second compound C2 in the second housing area HA2.

In particular, if the at least one containment element 4 advantageously comprises at least one containment portion 4a of the at least one second compound C2 and at least one abutment portion 4b with the second body 3a, in the folding operating configuration FC, the folding of the second body 3a, if at the folding means 5 or, advantageously, at the at least one weakening element or portion 6, brings the abutment portion 4b into abutment with the second body 3a or, more particularly with the receiving section 3e, and the pressure generated by this abutment causes the breaking of the at least one containment element 4 or, more particularly, of the narrowing 4c, at or close to the breaking zone or point BZ.

Thus, for example, with particular reference to the non-limiting embodiment shown in figure 14, the folding of the second body 3a by the operator, for example at the weakening portions 6, brings closer and in abutment each pointed portion 4b of the two vials 4 with the inside of the receiving section 3e, and the pressure generated by this abutment determines the breaking of each vial 4 at or in the vicinity of the breaking zone or point BZ, for example delimited by a narrowing 4c interposed between the cylindrical body 4a and the pointed portion 4b.

As can be understood, this breaking of at least one containment element 4 allows a clean cut of the same, thus avoiding its fragmentation.

As regards the release operating configuration REC, with reference by way of non-limiting example to figure 15, it is possible to speed up the release of the at least one second compound C2 from the vials 4, or in any case to facilitate its transfer from the second housing area HA2 to the first housing area HA1, using the pumping means 8 or, more particularly, the one or more enlarged or balloon-shaped portions 8a delimited in the second body 3a.

In this regard, the operator can consecutively press and release the pumping means 8 or, more particularly, the one or more enlarged or balloon-shaped portions 8a until the vials 4 have released all of the at least one second compound C2 in the second housing area HA2.

As regards, instead, the first component 2, it preferably comprises mixing means 10 configured, in use, to mix the at least one first compound C1 with the at least one second compound C2.

In particular, the mixing means 10 can advantageously comprise at least one handle component 11, a stem 12 which includes a first end 12a connected to the handle component 11 and a second end 12b, as well as a mixing component or rotor 13, connected to or supported by the second end 12b of the stem 12, designed, in use, to be translated and/or rotated inside the first housing area HA1 for mixing the at least two compounds C1, C2.

More in detail, the mixing component 13 can be connected to the stem 12 through a removable or non-removable engagement, for example through a bayonet or screw or similar engagement so that, as will be seen better below, it can be easily released from the stem 12, although this is not necessary.

According to the non-limiting embodiment of the present invention shown in the figures, the mixing component 13 can define a plurality of leaf portions 13a extending from a central zone CZ of the same.

In this regard, each leaf portion 13a can advantageously delimit a respective through hole TH which, together with the configuration of the leaf portion 13a is intended, in use, to improve the mixing of the mixture M.

As can be understood, this particular configuration of the mixing component 13 allows an efficient mixing of the at least two compounds C1, C2.

Clearly, this configuration is purely illustrative and not limiting.

The first component 2 preferably also comprises a pushing component or piston 14, slidably mounted in the first housing area HA1, and configured, in use, to push or press the mixture M out of the first housing area HA1, and locking means 15 of this pushing component 14 at one end of the first component 2 which is distal, in use, from the second component 3.

Basically, the locking means 15 can advantageously define with the at least one thrust component 14 at least two operating trims RD, TD.

These at least two operating trims RD, TD comprise at least one rest or locking operating trim RD, in which the locking means 15 are operatively associated with the pushing component 14 for locking the latter so that is not able to slide in the first housing area HA1 and at least one release or thrust operating trim TD, in which the locking means 15 are not operatively associated with the thrust component 14 so that the latter, in use, is able to slide and be pushed into the first housing area HA1.

As for the locking means 15, they may be or comprise a locking body 16 including at least one engaging or housing component or portion 16a, while the at least one pushing component 14 may comprise a base body 14a including at least one groove or lug 14b intended to house the engagement component or portion 16a or to be engaged in the housing component or portion 16a of the locking means 15.

In particular, the engaging component or portion 16a can have, for example, a substantially C or U-shaped plan configuration.

According to the non-limiting embodiment of the present invention shown in the figures, the engagement portion 16a of the locking means 15 has a substantially C-shaped plan configuration and is removably fitted or fittable in a respective groove 14b of the pushing component 14.

Furthermore, the first body 2a may comprise a flanged or enlarged end 2b opposite with respect to the connection end 2c.

In this case, the locking means 15 can advantageously comprise, on one proximal, in use, side to the connection end 2c, a first main wall section 16b, a side wall 16c which connects the first main wall section 16b to a second main wall section, which internally defines the engagement or housing portion 16a and defines with them an inserting zone IZ for at least part of the enlarged end 2b of the first body 2a.

If desired, the locking body 16 can also comprise or delimit a first terminal wall 16d, for example extending from the first main wall section 16b in the direction away from the inserting zone IZ, and configured, in use, to wrap part of the first body 2a and/or a second terminal wall 16e, for example extending from the engagement or housing component or portion 16a in the direction away from the inserting zone IZ, and configured, in use, to envelop at least part of the pushing component 14.

More specifically, with reference to the longitudinal axis of the first component 2, the locking means 15 can be removably engaged with the first main wall section 16b and the second main wall section perpendicular to the axis x-x and the side wall 16c coaxial to the axis x-x.

Basically, the locking means 15 or, more particularly, the locking body 16, can advantageously be removed from the pushing component 14 to make the latter pass from the rest operating trim RD to the thrust operating trim TD or on the contrary, it can be operatively associated or, more particularly, engaged or housed at least partially in the pushing component 14 to make the latter pass from the thrust operating trim TD to the rest operating trim RD.

This displacement or passage is obtained by relatively moving the first component of the locking means 15 in a transverse or orthogonal direction to the axis x-x. Basically, to release or constrain the locking means 15 and thus the pushing component 14 it is sufficient to move the locking means 15 transversely or orthogonally to the axis x-x, so as to determine the disengagement or engagement between the groove or lug 14b and the engagement component or portion 16a.

As it can be understood, in the rest operating trim RD the particular configuration of the locking means 15 or, more particularly, of the locking body 16 prevents the pushing component 14 from sliding into the first housing area HA1, thereby ensuring a considerable stability and sealing, for example during the mixing operations of the at least two compounds C1, C2 but also during the folding operations of the second body 3a and, if desired, also during the pumping operations of the second compound C2 in the second housing area HA2.

Entering into more detail of the exemplary and non-limiting configuration of the pushing component 14, it preferably delimits a through channel TC which defines at least two operating positions MP, CP for the mixing means 10.

These at least two operating positions MP, CP comprise at least one mixing operating position MP in which the stem 12 is slidably inserted into the through channel TC and at least one closing operating position CP in which the stem 12 is extracted from said through channel TC and the mixing component 13 is inserted, for example to size or by screwing or bayonet engagement, in the through channel TC.

Basically, during the mixing of the at least two compounds C1, C2 inside the first housing area HA1, the mixing means 10 are in the mixing operating position MP so that it is possible, for example for the operator, to grip the handle component 11 and slide the stem 12 inserted into the through channel TC so as to mix the at least two compounds C1, C2 through the mixing component 13 in order to obtain the mixture M.

Once the mixing has been carried out, the operator can make the mixing means 10 pass from the mixing operating position MP to the closing operating position CP, by extracting the stem 12 from the through channel TC, if desired to the end of its stroke, and subsequently rotating the handle component 11, for example in an anti-clockwise direction, so as to release the stem 12 or better the end 12b thereof from the mixing component 13 removably engaged with it.

In this way, the mixing component 13 remains advantageously engaged, for example to size or by screwing or bayonet engagement, in the through channel TC, thus ensuring the fluid tightness of the first housing area HA1.

Preferably, the first component 2 or, more particularly, the first body 2a comprises a connection end 2c which is, in use, proximal to the second component 3, for example a threaded end, and at least one closure cap 18 configured, in use, to be operatively associated, for example by screwing, to said end 2c so as to close the first body 2a.

According to the non-limiting embodiment of the present invention shown in figure 1, the second component 3 is operatively associated with the first component 2 by means of a threaded coupling (or of another type, such as a bayonet coupling, fitting, etc.) of the end 2c of the first component 2 with the end 3b internally threaded of the second component 3.

So far as the closure cap 18 is concerned, it can comprise or delimit at least one through duct 18b, in use, in fluid communication with the first housing area HA1. If desired, the closure cap 18 can be designed to be placed in fluid communication with suction means of the air present inside the first housing area HA1.

The closure cap 18 can be operatively associated with the end 2c of the first body 2a, for example before mixing the at least two compounds C1, C2, after having disassembled or unscrewed the second component 3.

In particular, if the first body 2a has a threaded end 2c, the closure cap 18 will also have a threaded end 18a, in order to allow an easy connection between them.

The suction means are capable of generating a pressure reduction inside the first housing area HA1 and can be operated, for example during the mixing of the at least two compounds C1, C2, in order to reduce the formation of bubbles of the mixture M and increase its properties.

The suction means may include, for example, devices operated by a motor, if desired electric or according to the Venturi effect. Alternatively, the mixing device 1 can be placed in fluid communication with a centralized vacuum line provided, for example, in operating rooms.

Clearly, the suction means may also not be used or provided.

Furthermore, the mixing device 1 can advantageously also comprise sealing means 19, for example one or more gaskets 19, if desired positioned in a respective annular seat 14c of the pushing component 14 and/or positioned in the through channel TC of the pushing component 14, designed to ensure the fluid tightness of the first housing area HA1 at the pushing component 14.

According to the non-limiting embodiment of the present invention shown in the figures, a first gasket 19a is provided in the through channel TC so that fluid tightness is ensured when the rod 12 is moved or, more particularly, made to slide at the inside the through channel TC for mixing the at least two compounds C1, C2, and a second gasket 19b positioned in a respective annular seat 14c delimited by the pushing component 14 so that the fluid tightness is guaranteed also at the edges 14d of the pushing component 14 of one of its internal, in use, faces IF to the first housing area HA1.

A brief non-limiting example of operation of the present invention is described below.

We will assume that the entire process of obtaining the M mixture is carried out by an operator, for example a surgeon assigned to the preparation of a prosthesis or a joint spacer. Clearly in this case the mixture M will preferably be bone cement for medical use.

Initially, the first component 2 is advantageously closed by the closure cap 18, if provided, to prevent environmental dust or in any case foreign bodies from entering and polluting the first compound C1 present in the first housing area HA1.

The operator can then remove the closure cap 18, for example by unscrewing it, and operatively associate, for example by screwing, the second component 3 to the first component 2. The mixing device 1 is thus in the rest operating configuration RC.

The operator can then move or incline the containment element 4 transversely or orthogonally to the transfer axis or direction AD between a first rest position (see figure 1) in which a respective abutment portion is not in contact with or breaking or opening thrust against the second component 3 or with elements of the device integral with it and at least a second breaking or opening position (see figure 14) in which the abutment portion impacts against the second component 3 or presses against it or with an element of the device integral with it to a greater extent than in the first position.

This can be achieved for example by grasping the second body 3a, for example with a hand at the connection end 3b of the second component 3 or at the end 2c of the first component 2, and folding or moving it at a respective portion or section 3c, 3d, 3f.

The particular configuration of the second body 3a, with the possible folding means 5 or, more particularly, with the possible weakening elements or portions 6 or, even more in detail, with the depressions 6 and the stiffening elements or sections 7, allow the folding of said second body 3a, for example at these depressions 6. When doing so, the abutment portion 4b or, more particularly, the tip 4b of the containment elements 4, contacts a component or element or portion of the second component 3, such as an internal wall section of section 3e and the pressure generated by this abutment causes the breakage of the containment elements 4 or, more particularly, of the vials 4 at or in the vicinity of the breaking zone or point BZ delimited, for example, by the narrowing 4c, if provided. The mixing device 1 is therefore in the folding operating configuration FC.

In this folding operating configuration FC the second compound C2 is slowly released from the containment elements 4 since the air can enter them, however, as can be understood, it can be inconvenient for the operator to remain with the second body. 3a folded in order to release all of the second compound C2.

In this regard, the operator can, if the second body 3a is made of elastic or plastic material, release the folding or displacement or inclination of the latter so that the mixing device 1 passes into the release operating configuration REC.

In this release operating configuration REC, it is possible to exploit the pumping means 8, if provided, or, more particularly, the one or more enlarged or balloon-shaped portions 8a delimited in the second body 3a to release more quickly or in any case facilitate the transfer of the second compound C2 from the second housing area HA2 to the first housing area HA1. In particular, the operator can press and subsequently release, if desired consecutively, the one or more enlarged or balloon-shaped portions 8a so as to decrease the air pressure inside the second housing area HA2 which thus allows the pressure of the air contained in the containment element 4 and the hydrostatic pressure of the second compound 2 to overcome the pressure acting, in use, on the breaking zone or point BZ, effectively releasing the second compound C2 in the second housing area HA2 more quickly.

The second compound C2, through the through opening TO delimited in the connection end 3b, can thus flow from the second housing area HA2 to the first housing area HA1. The broken parts, i.e., the abutment portions 4b of the containment elements 4 are instead retained in the second housing area HA2, if desired by the retaining elements 3h positioned at the through opening TO.

At this point the operator can disassociate, for example by unscrewing, the second component 3 from the first component 2 and re-apply the closure cap 18, if desired by connecting operatively the through duct TD to the suction means, for example for generating a vacuum inside the first housing area HA1, to then proceed with the mixing of the two compounds C1, C2 by means of the mixing means 10.

Alternatively, if the operator does not want to create a vacuum, he can proceed to mix the two compounds C1, C2 by means of the mixing means 10 while maintaining the second component 3 operatively associated with the first component 2.

In particular, to mix the two compounds C1, C2 present in the first housing area HA1, the operator can grasp the handle component 11 to slide the stem 12 and therefore also the mixing component 13, connected or in any case supported by the second end 12b of the stem 12 itself, in the through channel TC of the pushing component 14. Therefore, the mixing component 13 can translate and/or rotate in the first housing area HA1, actually mixing the two compounds C1, C2 so as to obtain a mixture M. Before and during mixing, the mixing means 10 are in the mixing operating position MP while the pushing component 14 and the locking means 15 are in the rest operating trim RD.

Once the mixing of the two compounds C1, C2 has been completed, the operator can, if he has carried out this mixing with the second component 3 still operatively associated with the first component 2, disassociate such second component 3 and apply the closure cap 18 at the end 2c of the first component 2, for example by screwing.

Subsequently, according to a possible but not necessarily present variant, the operator can grasp the handle component 11 to extract the stem 12 from the through channel TC, for example by bringing it to the end of its stroke and then rotating it, if desired, in an anti-clockwise direction, so as to release the end 12b of the stem 12 from the mixing component 13, which remains engaged, for example to size in the through channel TC, by making the mixing means 10 pass from the mixing operating position MP to the closing operating position CP.

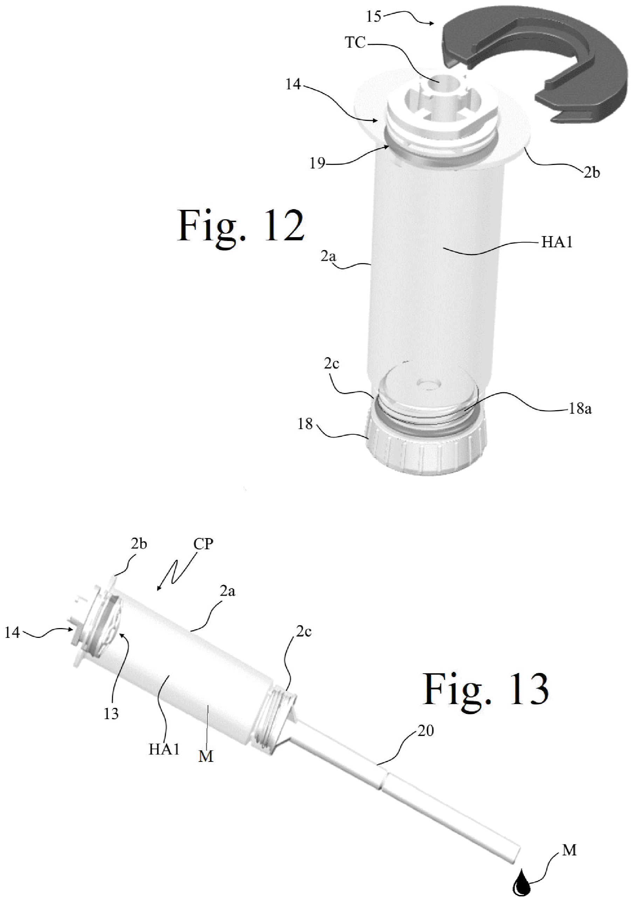

The operator can then remove the closure cap 18 again and connect to the end 2c of the first component 2 of the expulsion means 20 of the mixture M previously obtained, for example a special cannula 20, visible according to a non-limiting embodiment in figure 13.

Subsequently, the operator can remove the locking means 15 or, more particularly, the locking body 16, if desired having a substantially C-shaped plan configuration, so that the pushing component 14 passes from the rest operating trim RD to the thrust operating trim TD.

Finally, the operator can mount or in any case connect to the base body 14a of the pushing component 14 extrusion means, for example a pneumatic gun, intended to push the pushing component 14 which in turn, being in the thrust operating trim TD, can slide within the first housing area HA1 to extrude or make the mixture M come out, for example in the expulsion means or in any case outside the first housing area HA1.

Of course, if a pneumatic gun is used, it will be advantageously compatible to be connected with the base body 14a of the pushing component 14.

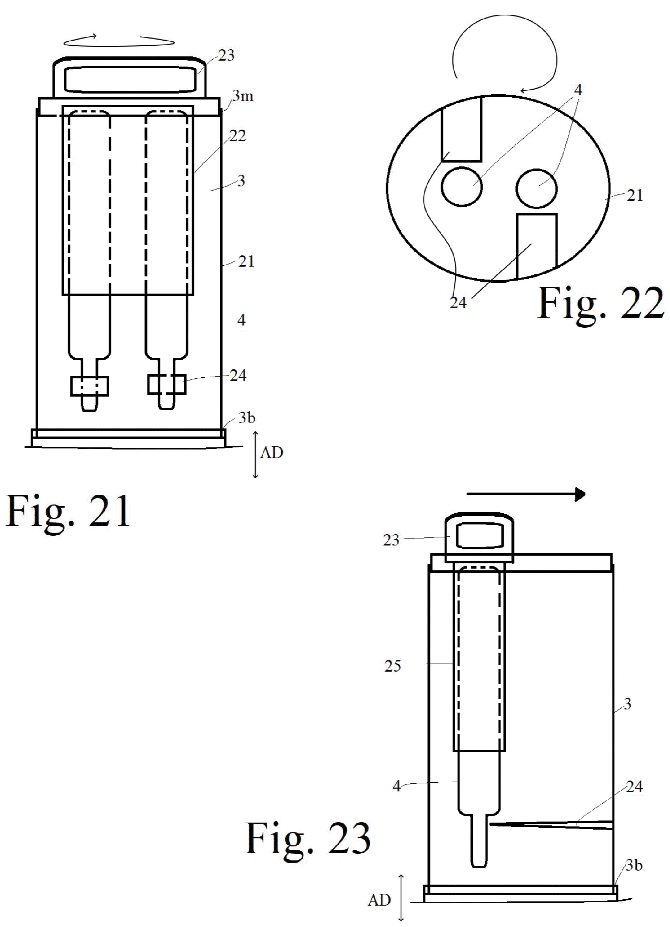

With reference now to figures 21 and 22, a device similar to the one described above has been illustrated, but the second body 3a is in two pieces, a first outer piece 21 tubular or incorporating or defining the constraint end 3b with a second end 3m opposite to the constraint end 3b, that is open. A second piece 22 of the body 3a is then rotatably mounted in the second end 3m, which piece could advantageously have a handle or grip 23 incorporated or in one piece therewith.

The containment elements 4 would in this case be mounted on the second piece 22, so that they would be rotatably mounted, if desired by means of the handle 23 with respect to the first piece 21.

In this case, the device 1 could also comprise inside the second piece 22, if desired in one piece or integral with it, one or more wall or knife portions 24 designed to intercept and break the containment elements 4, for example at the breaking or opening zone or point BZ, when the latter are made to rotate together with a respective second piece 22.

With such a device, in order to break or open the containment elements 4, one should, by means of a suitable handle 23, determine the rotation of the second revolving piece 22 and therefore of the containment elements 4, thus causing the latter to impact, if desired, the respective tip with a respective wall or knife portion 24, thereby opening the containment elements 4.

In this case, the expression movable or inclinable transversely or orthogonally is in this case interpreted in the sense that the containment element 4 is movable by rotation around the transfer axis or direction between the first rest position and the second breaking or opening position.

In accordance with another variant shown in figure 23, the containment elements 4 are mounted sliding (and not rotating) by means of a respective slide and shoe engagement with the second component 3.

In this regard, the second end 3m of the second component 3 opposite the constraint end 3b defines at least one seat or groove in which the containment elements 4 or a respective support 25 are sliding mounted.

Subject-matter of the present invention is also a method of mixing at least two compounds C1, C2 to obtain a mixture M.

This method initially comprises the step of placing at least a first compound C1, for example a powder, such as one or more polymers, eventually acrylic, in a first housing area HA1 delimited by a first component 2 comprising a first body 2a.

Subsequently, it is provided the step of inserting at least one containment element 4, for example at least one vial, containing at least a second compound C2, if desired a liquid, such as a monomer, into a second housing area HA2 delimited by a second component 3 comprising a second body 3a.

The method according to the present invention then includes the step of operatively associating the second component 3 to the first component 2, for example by screwing or bayonet coupling or a similar means. Clearly, the two components 2 and 3 can be associated even before the positioning of at least one containment element 4.

Then there is the step of moving or tilting the containment element 4 transversely or orthogonally to the transfer axis or direction between a first rest position and at least a second position.

This can for example be obtained by folding the second body 3a, if desired at the folding means 5, for example at least one weakening element or portion 6 of the second body 3a, so as to determine the displacement or folding of the at least one containment element 4 integral in displacement with the body 3a and therefore the breaking of the at least one containment element 4 following impact against a portion of the body 3a or of the device, thereby releasing the at least one second compound C2 so that the latter last passes or flow from the second housing area HA2 to the first housing area HA1.

Preferably, following the step described above, the method provides for the step of consecutively pressing and releasing the second body 3a, if desired at the pumping section 3f so as to speed up the release of the at least one second compound C2 from the at least one containment element 4.

The method according to the present invention then provides for the step of mixing in the first housing area HA1 the at least one first compound C1 with the at least one second compound C2 so as to obtain a mixture M.

Thereafter, the step of separating the second component 3 from the first component 2 can be envisaged before or after the aforementioned mixing step, for example by unscrewing.

In the event that the disassociation step was arranged before the mixing phase, following this separating step, the step of sucking, by means of suction means, the air present inside the first housing area HA1 so as to create a vacuum in it, for example in order to reduce the formation of bubbles of the mixture M and increase its properties can be advantageously provided.

Finally, the step of extruding or pushing the previously obtained mixture M out of the first housing area HA1 is envisaged, for example by means of extrusion means, such as a pneumatic gun.

This step of extruding can comprise the sub-step of operatively connecting extrusion means to the first component 2.

Moreover, the step of extruding can also comprise the sub-step of operatively connecting expulsion means 20 of the mixture M previously obtained to the first component 2, for example a cannula 20, so as to facilitate its expulsion.

The method according to the present invention can be advantageously implemented or performed by means of a mixing device 1 according to the present invention or even more advantageously by means of a mixing device 1 according to the non-limiting example of embodiment shown in the figures.

In particular, if made or carried out by means of a mixing device 1 according to the non-limiting example of embodiment shown in the figures, the step of pressing and releasing is preferably carried out by means of pumping means 8 or, more particularly, by means of the one or more enlarged or balloon-shaped portions 8a delimited in the second body 3a.

Furthermore, again if carried out by means of a mixing device 1 according to the non-limiting example of embodiment shown in the figures, the mixing step is preferably carried out by means of the mixing means 10, for example by grasping the handle component 11 to slide, inside of the through channel TC delimited by the pushing component 14, the stem 12 and consequently also the mixing component 13 which can therefore translate and/or rotate in the first housing area HA1, effectively mixing the two compounds C1, C2 so as to obtain a mixture M.

Following the mixing step, the step of extracting the stem 12 of the mixing means 10 from the through channel TC, if desired to the end of its stroke, can also be advantageously provided, and then rotate the handle component 11, for example anti-clockwise, so as to release the stem 12 or, more particularly, the end 12b thereof, from the mixing component 13 removably engaged with it.

Furthermore, always considering that the method is carried out by means of a mixing device 1 according to the non-limiting example of embodiment shown in the figures, before the step of extruding, is preferably provided the step of extracting or removing the locking means 15 or, more in detail, the locking body 16a, for example to be able to easily associate the extrusion means with the pushing component 14.

As can be understood, the mixing device 1 as well as the method according to the present invention are easy and intuitive to use.

Furthermore, with respect to the mixing devices taught by the state of the art, the mixing device 1 according to the present invention has fewer components and therefore lower costs, as well as a considerably lower structural complexity.

Moreover, the mixing device 1 according to the present invention allows rapid and effective mixing of the compounds.

Furthermore, the breaking of the at least one containing element 4 for the release of the at least one second compound C2 occurs by clean cut or in any case not as foreseen according to the state of the prior art, preferably avoiding the fragmentation of the containing element 4 and consequently, also the accidental fall of fragments inside the first housing area HA1 where the mixing of the at least two compounds C1, C2 will take place.

It should be noted that the transfer of at least one second compound C2 and also its subsequent mixing with the at least one first compound C1 always takes place in a closed environment, and therefore safe from any external agents that could compromise the use of the mixture M.

It should also be considered that the mixing device 1 according to the present invention is very versatile, as it can be operatively associated, without further need for adaptation components, with extrusion and/or expulsion means to facilitate the discharge of the mixture M from the first housing area HA1.

It has thus been seen that the present invention fully achieves the proposed objects.

The mixing device 1 as well as the method described above are susceptible of numerous modifications and variations within the scope of protection of the following claims.

Furthermore, all the details can be replaced by other technically equivalent elements.

In practice, the shapes and the contingent dimensions may be any according to requirements without thereby abandoning the scope of the protection of the following claims.

## Claims

1. Device for mixing (1) at least two compounds (C1, C2) for obtaining a mixture (M), comprising:
- a first component (2) including a first body (2a) defining a first housing area (HA1) for at least one first compound (C1);
- a second component (3), in use, operatively associable with said first component (2) so as to place said first and said second component (2, 3) in fluid communication defining a fluid transfer axis (AD) from said first (2) to said second (3) component, said second component (3) including a second body (3a) defining a second housing area (HA2);
- at least one element for containing (4) at least one second compound (C2), positioned or placeable within said second housing area (HA2), said at least one containment element (4) comprising or delimiting at least one breaking or opening zone or point (BZ);
said at least one containment element (4) being displaceable or inclinable transversely or orthogonally to said transfer axis (AD) between a first rest position in which a respective abutment portion (4b) is not in contact with or in thrust such to determine its breaking or opening against said second component (3) or with elements of said device integral with it and at least one second breaking or opening position in which said abutment portion (4b) impacts against said second component (3) or presses against the same or with an element of said device integral therewith to a greater extent than said first position, so that when passing from said first to said at least one second position said at least one containment element (4) breaks or opens in such a way that the at least one second compound (C2) inside it passes or flows from said second housing area (HA2) to said first housing area (HA1) along said fluid transfer axis (AD),
wherein said second body (3a) is made of thermoplastic polyurethane or TPU and said second component (3) is wholly or partly translucent or transparent.

2. Device according to claim 1, wherein said second component (3) has a constraint or connection end (3b) to said first component (2) defining or extending around said fluid transfer axis (AD) and at least one projecting section (3c, 3d, 3f) from said constraint or connection end (3b) and inclinable or foldable with respect to said fluid transfer axis (AD), said at least one containment element (4) being integral in displacement or inclination with said at least one projecting section (3c, 3d, 3f), so that, by determining the inclination or folding of said at least one projecting section (3c, 3d, 3f) with respect to said constraint or connection end (3b), the inclination of said at least one containment element (4) mounted within said second component (3) and therefore the passage of the same between said first and said at least one second position and the respective breaking or opening thereof is determined.

3. Device according to claim 1 or 2, **characterized in that** said second component (3) comprises folding means (5) configured to allow or facilitate the folding of at least one portion or section (3c, 3d, 3f) of said second body (3a) from said first to said at least one second position, so as to determine the breaking or opening of said at least one containment element (4) at or around said breaking or opening zone or point (BZ) so that the at least one second compound (C2), in use, passes or flows from said second housing area (HA2) to said first housing area (HA1).

4. Mixing device (1) according to the preceding claim, wherein said folding means (5) are or comprise at least one weakening portion or element (6) delimited in said second body (3a) so that, following the folding of the latter, it folds at said at least one weakening portion or element (6) to cause the breakage or opening of said at least one containment element (4) at or around said breaking or opening zone or point (BZ).

5. Mixing device (1) according to claim 4, wherein said at least one weakening portion or element (6) consists of two, three, four or more depressions (6) delimited in said second body (3a) and positioned staggered with respect to each other.

6. Mixing device (1) according to any one of the preceding claims, wherein said second component (3) comprises or delimits one or more stiffening elements or sections (7) configured to stiffen said second body (3a) so that the latter, in use, does not fold at said one or more stiffening elements or sections (7).

7. Mixing device (1) according to any one of the preceding claims, wherein said second component (3) has, from one end to the other, several tubular sections, one with a section different from the others designed to internally define a specific internal face or surface defining the second housing area (HA2), said sections comprising at least one section for holding (3c, 3d) said at least one containment element (4) designed to clamp or enclose a respective part of said at least one containment element (4) so as to keep it in a specific relative position with respect to said second component or to a connection end (3b) of the same and at least one receiving section (3e) with slack or play of a respective abutment portion (4b) of said at least one containment element (4).

8. Mixing device according to claim 7, wherein said at least one holding section (3c, 3d) is internally defined by respective projections or protuberances or recesses (3c1, 3d1) designed to engage a respective portion of said at least one containment element (4).

9. Mixing device according to claim 7 or 8, wherein said receiving section (3e) has an internal section greater than a respective portion (4b) of said at least one containment element (4) or in any case the portion (4b) of said at least one containment element (4) arranged inside the area or zone defined by said at least one receiving section (3e) is not clamped by the latter and is free to move or incline with respect to said at least one receiving section (3e) in the passage from the first to the second position.

10. Mixing device according to claim 7 or 8 or 9, wherein said second body (3a) comprises a bellows element or section (3i) arranged between said at least one holding section (3d) and said at least one receiving section (3e), said at least one bellows element or section (3i) being designed to facilitate the folding of said second body (3a) so as to simplify or in any case improve the process of obtaining the mixture (M).

11. Mixing device (1) according to any one of the preceding claims, wherein said second component (3) comprises or delimits at least one positioning seat (PS) configured to support said at least one containment element (4).

12. Mixing device (1) according to the preceding claim, wherein said at least one containment element (4) is engaged in said at least one positioning seat (PS) by means of forced or to size fitting.

13. Mixing device (1) according to any one of the preceding claims, wherein said second component (3) comprises or delimits a constraint or connection end (3b) to said first component (2), said constraint or connection end (3b) defining at least one through opening (TO) so as to allow, in use, the passage or transfer of said at least one second compound (C2) from said second housing area (HA2) to said first housing area (HA1).

14. Mixing device (1) according to any one of the preceding claims, wherein said first component (2) comprises mixing means (10) configured, in use, to mix said at least one first compound (C1) with said at least one second compound (C2).

15. Mixing device (1) according to any one of the preceding claims, wherein said first component (2) comprises at least one pushing component or piston (14), slidably mounted in said first housing area (HA1), and configured, in use, for pushing or pressing the mixture (M) out of said first housing area (HA1) and locking means (15) of said pushing component (14) at one end of the first component (2) which is distal, in use, from the second component (3).

16. Mixing device (1) according to the preceding claim, wherein said locking means (15) define with said at least one pushing component (14) at least two operating trims (RD, TD), said at least two operating trims (RD, TD) comprising at least one rest or locking operating trim (RD) in which said locking means (15) are operatively associated with said pushing component (14) for locking the latter so that is not able to slide in said first housing area (HA1) and at least one release or thrust operating trim (TD) in which said locking means (15) are not operatively associated with said pushing component (14), so that the latter, in use, is able to slide and be pushed into said first housing area (HA1).

17. Mixing device (1) according to claim 15 or 16, wherein said locking means (15) are or comprise a locking body (16) including at least one engaging or housing component or portion (16a) and wherein said at least one pushing component (14) comprises a base body (14a) including at least one groove or lug (14b) intended to house said engaging component or portion (16a) or to be engaged in said housing component or portion (16a).
